# EUROPEAN PATENT SPECIFICATION

(11) **EP 3 778 879 B1**
(45) Date of publication and mention of the grant of the patent: **22.07.2026**
(21) Application number: 19774786.8
(22) Date of filing: 26.03.2019
(51) Int. Cl.: C12N 5/0793, A61K 35/30, A61P 21/00, C12N 5/071

(54) **METHOD FOR DIFFERENTIATING MOTOR NEURONS FROM TONSIL-DERIVED MESENCHYMAL STEM CELLS**
VERFAHREN ZUR DIFFERENZIERUNG VON MOTORISCHEN NEURONEN AUS MESENCHYMALEN STAMMZELLEN DER TONSILLEN
MÉTHODE POUR LA DIFFÉRENCIATION DE NEURONES MOTEURS À PARTIR DE CELLULES SOUCHES MÉSENCHYMATEUSES DÉRIVÉES DE TONSIL

(30) Priority: 26.03.2018 KR 20180034779
(43) Date of publication of application: 17.02.2021
(73) Proprietor: EHLBIO Co., Ltd., 16006 Gyeonggi-do (KR)
(72) Inventor: PARK, Saeyoung, Seoul 07005 (KR); JUNG, Sung Chul, Seoul 06712 (KR); MYUNG, Seo-ha, Seoul 06347 (KR); JUNG, Soo Yeon, Seoul 04205 (KR); KIM, Jiyeon, Seoul 07792 (KR); JUNG, Namhee, Seoul 07270 (KR); CHOI, Yeonzi, Seoul 04502 (KR)
(74) Representative: V.O.
(86) International application number: PCT/KR2019/003520
(87) International publication number: WO 2019/190175

(56) References cited:
- KR-A- 20140 126 626
- KR-A- 20160 086 170
- KR-B1- 101 717 402
- QU QIUHAO ET AL: "High-efficiency motor neuron differentiation from human pluripotent stem cells and the function of Islet-1", vol. 5, no. 1, 13 March 2014 (2014-03-13), XP055857870, Retrieved from the Internet <URL:http://www.nature.com/articles/ncomms4449> DOI: 10.1038/ncomms4449
- PATEL MADHUMITA ET AL: "Microsphere-Incorporated Hybrid Thermogel for Neuronal Differentiation of Tonsil Derived Mesenchymal Stem Cells", ADVANCED HEALTHCARE MATERIALS, vol. 4, no. 10, 1 June 2015 (2015-06-01), DE, pages 1565 - 1574, XP055857939, ISSN: 2192-2640, DOI: 10.1002/adhm.201500224
- PARK S ET AL: "Neural progenitors generated from the mesenchymal stem cells of first-trimester human placenta matured in the hypoxic-ischemic rat brain and mediated restoration of locomotor activity", PLACENTA, W.B. SAUNDERS, GB, vol. 32, no. 3, 25 December 2010 (2010-12-25), pages 269 - 276, XP028148747, ISSN: 0143-4004, [retrieved on 20110110], DOI: 10.1016/J.PLACENTA.2010.12.027
- BAGHER, Z.: "Induction of human umbilical Wharton's jelly-derived mesenchymal stem cells toward motor neuron-like cells", IN VITRO CELLULAR & DEVELOPMENTAL BIOLOGY-ANIMAL, vol. 51, 2015, pages 987 - 994, XP035574273, DOI: 10.1007/s11626-015-9921-z
- JUNG, N.: "Tonsil-derived mesenchymal stem cells differentiate into a schwann cell phenotype and promote peripheral nerve regeneration", INTERNATIONAL JOURNAL OF MOLECULAR SCIENCES, vol. 17, 1867, 2016, XP055693202
- ABDULLAH, R. H.: "Induction of mice adult bone marrow mesenchymal stem cells into functional motor neuron-like cells", JOURNAL OF CHEMICAL NEUROANATOMY, vol. 77, 2016, pages 129 - 142, XP029753227, DOI: 10.1016/j.jchemneu.2016.07.003
- BAGHER, Z.: "Differentiation of wharton' s jelly-derived mesenchymal stem cells into motor neuron-like cells on three-dimensional collagen-grafted nanofibers", MOLECULAR NEUROBIOLOGY, vol. 53, 2016, pages 2397 - 2408, XP035953149, DOI: 10.1007/s12035-015-9199-x
- PARK, GYEONGHO ET AL.: "EO-61 Differentiation Induction of Neurons from Tonsil Mesenchymal Stem Cells", THE 16TH COMBINED CONFERENCE OF OTORHINOLARYNGOLOGY HEAD AND NECK SURGERY, 29-30 OCTOBER 2010, CHANGWON EXHIBITION CONVENTION CENTER (KOREA), vol. 16, 29 October 2010 (2010-10-29), pages EO-61, XP009523781

## Description

### [Technical Field]

The present disclosure relates to a method for differentiating motor neurons from tonsil-derived mesenchymal stem cells, and a cell therapy agent using the same.

### [Background Art]

Stem cells are cells that can differentiate into various types of cells constituting the tissues of an organism and collectively refer to undifferentiated cells that can be obtained from the tissues of embryonic, fetal and adult organisms. Stem cells are characterized in that they differentiate into specific cells in response to differentiation stimuli (environments), can produce the same cells through cell division (self-renewal) and have the plasticity to differentiate into different cell types depending on differentiation stimuli.

Stem cells can be classified into pluripotent stem cells, multipotent stem cell and unipotent stem cells based on their differentiation potency. Pluripotent stem cells are cells having the pluripotency of capable of differentiating into any cell type, whereas some stem cells have multipotency or unipotency.

Since the stem cells are applicable as a cell therapy agent based on their differentiation potency, research and development are being carried out actively in this regard. However, a cell therapy agent using embryonic stem cells raises ethical concerns or tissue compatibility problems, and use of induced pluripotent stem cells as a cell therapy agent has the problem of tumor formation.

Therefore, researches are being conducted using mesenchymal stem cells, which have lower differentiation potency but are relatively safe. Mesenchymal stem cells (MSCs) refer to multi-potential non-hematopoietic precursor cells in the bone marrow, etc. of adults, which can differentiate into a variety of cell types such as adipocytes, cartilage cells, bone cells, muscle cells, skin cells, etc. Clinical researches are being conducted on regeneration of various tissues using the mesenchymal stem cells and they also show applicability in organ transplantation.

However, some of the mesenchymal stem cells are restricted in applications because it is difficult to obtain them. For example, the cells that can be obtained through the most non-invasive method are mesenchymal stem cells obtained from bone marrow. But, the non-invasive bone marrow harvesting requires anesthesia, induces pain and is restricted in applications. To solve this problem, it is necessary to acquire cells from peripheral blood in order to isolate patient-specific stem. However, the number of mesenchymal stem cells that can be isolated from adult peripheral blood is too small and the isolation method is not economical. And, even if they are isolated, it is not easy to proliferate them to an amount applicable for cell therapy. Therefore, a more practicable alternative method is necessary.

In addition, adult stem cells obtained from elderly patients exhibit significantly reduced proliferative ability as well as decreased ability of secreting various factors, migrating to lesions, etc. as compared to the cells obtained from younger patients. Therefore, it is necessary to obtain cells from the tissues that can be isolated naturally or discarded from younger patients.

Meanwhile, research and development are being conducted on treatment of diseases associated with congenital or genetic loss of motor neurons or acquired loss of nerve tissues using stem cells. For example, it was reported that transplantation of neural stem cells wherein vascular endothelial growth factors are overexpressed to an animal model of Lou Gehrig's disease resulted in delayed onset of Lou Gehrig's disease and significant improvement in motor function. In addition, researches are being carried out on a method of treating Lou Gehrig's disease by transplanting stem cells extracted from fat or bone marrow together with vascular endothelial growth factors. But, vascular endothelial growth factor cannot cross the blood-brain barrier due to a very large size and is lost in short time due to short half-life. Therefore, the therapeutic effect of a stem cell therapy agent using the vascular endothelial growth factor is very limited.

Accordingly, there is a need of research and development on a method for securing motor neurons suitable for application to the human body by identifying an optimum differentiation method capable of ensuring high differentiation potency from specific stem cells into motor neurons.

### [References of Related Art]

### [Patent Documents]

(Patent document 1) International Patent Publication No. WO2017/135753.

### [Disclosure]

### [Technical Problem]

The present disclosure relates to a differentiation medium composition suitable for differentiating tonsil-derived mesenchymal stem cells or neural precursor cells differentiated therefrom into motor neurons, comprising low-glucose DMEM (Dulbecco's modified Eagle's medium), FBS, N₂ supplement, retinoic acid, brain-derived neurotrophic factor (BDNF), nerve growth factor (NGF) and sonic hedgehog (SHH).

The present disclosure also relates to a method for differentiating into motor neurons using the differentiation medium composition.

The present disclosure also relates to motor neurons prepared by the method.

The present disclosure also relates to a pharmaceutical composition for preventing or treating a neurological disorder, which contains the motor neurons.

### [Technical Solution]

The inventors of the present disclosure have researched method for producing motor neurons applicable to the human body in large scale and have completed the present disclosure by inventing a method for producing motor neurons from tonsil-derived mesenchymal stem cells in large scale in short time.

In an aspect, the present disclosure provides a differentiation medium composition suitable for differentiating stem cells or neural precursor cells into motor neurons, which contains low glucose DMEM (Dulbecco's modified Eagle's medium), FBS, N₂ supplement, retinoic acid, brain-derived neurotrophic factor (BDNF), nerve growth factor (NGF) and sonic hedgehog (SHH).

Specifically, the differentiation medium used for inducing differentiation into motor neurons may contain low-glucose DMEM, 0.25-25% (w/v) FBS, 0.1-10% (w/v) N₂ supplement, 0.1-10 µM retinoic acid, 1-100 ng/mL brain-derived neurotrophic factor, 1-100 ng/mL nerve growth factor and 0.01-1 ng/mL sonic hedgehog. Most specifically, it may contain low-glucose DMEM, 2.5% (w/v) FBS, 1% (w/v) N₂ supplement, 1 µM retinoic acid, 10 ng/mL brain-derived neurotrophic factor, 10 ng/mL nerve growth factor and 0.1 ng/mL sonic hedgehog.

Although high-glucose DMEM (Dulbecco's modified Eagle's medium) is used in a differentiation medium in general, the use of low-glucose DMEM as in the present disclosure can improve the efficiency and directionality of differentiation by stopping the proliferation or growth of cells and creating a minimum environment for survival of cells (for initiating differentiation).

In the differentiation medium, the N₂ supplement can induce the initiation of specific differentiation into motor neuron because it is free from biotin, L-carnitine, corticosterone, ethanolamine, D(+)-galactose, glutathione (reduced), linolenic acid, linoleic acid, retinyl acetate, selenium, T3 (triodo-1-thyronine), D/L-α-tocopherol (vitamin E), D/L-α-tocopherol acetate, catalase, superoxide dismutase, etc. contained in B27 supplement.

In the differentiation medium, the brain-derived neurotrophic factor (BDNF) refers to a neurotrophic factor mainly found in the brain, which is involved in the development, growth, function, neuroplasticity, etc. of neuronal cells.

In the differentiation medium, the nerve growth factor (NGF) refers to a cytokine peptide factor involved in the differentiation and growth of nerve tissues.

The differentiation medium of the present disclosure contains low-glucose DMEM and FBS unlike the media commonly used in proliferation of stem cells, and exhibits remarkable effect as compared to the existing stem cell culture media. In particular, the differentiation medium used for induction of motor neurons of the present disclosure contains all of low-glucose DMEM, FBS, N₂ supplement, retinoic acid, brain-derived neurotrophic factor (BDNF), nerve growth factor (NGF) and sonic hedgehog (SHH), and exhibits remarkable effect of differentiating into motor neurons as compared to the media lacking some of the ingredients.

In another aspect, the present disclosure provides a method for differentiating tonsil-derived mesenchymal stem cells or neural precursor cells into motor neurons, which includes a step of inducing differentiation into motor neurons by culturing tonsil-derived mesenchymal stem cells or precursor cells differentiated therefrom in the differentiation medium composition described above, wherein, in case of differentiating tonsil-derived mesenchymal stem cells into motor neurons, the method further comprises culturing the tonsil-derived mesenchymal stem cells in a suspended state to form cell aggregates and a step of differentiating the formed cell aggregates into neural precursor cells by subculturing up to 1 -3 passages.

In the differentiation method of the present disclosure, the culturing may be performed specifically for 2-4 weeks.

In the present disclosure, the motor neurons refer to neurons whose neurites govern skeletal muscles. They are also called motoneurons and are present mainly in the motor field of the cerebral cortex and the anterior horn of the spinal cord. Specifically, the cells up to the anterior horn of the spinal cord are called upper motor neurons (motoneurons), and the cells below the anterior horn of the spinal cord are called lower motor neurons (motoneurons).

In the present disclosure, the tonsil-derived mesenchymal stem cells refer to undifferentiated stem cells derived from the tonsils, which are epithelial lymphoid tissues located inside the throat and behind the nose that primarily defend our bodies against bacteria, etc. invading from outside, having the ability of self-renewal and differentiation into two or more types of cells.

According to a specific exemplary embodiment of the present disclosure, the tonsil-derived mesenchymal stem cells exhibit higher expression of the neural precursor cell marker vimentin as compared to mesenchymal stem cells derived from other tissues.

Specifically, the mesenchymal stem cells derived from other tissues include adipose-derived mesenchymal stem cells (AdMSC), bone marrow-derived mesenchymal stem cells (BM-MSC) and umbilical cord-derived or cord blood-derived mesenchymal stem cells (e.g., Wharton's jelly-derived mesenchymal stem cells (WJ-MSC)), although not being limited thereto. The tonsil-derived mesenchymal stem cells exhibit higher expression of the neural precursor cell marker vimentin by 10% or more, specifically 30% or more, as compared to the mesenchymal stem cells derived from other tissues.

In the present disclosure, the precursor cell refers to a cell in a stage before having the morphology and function of a specific type of cell. Specifically, a neural precursor cell refers to a precursor cell that can differentiate into neurons, astrocytes, oligodendrocytes, etc. constituting the central nervous system.

According to a specific exemplary embodiment of the present disclosure, the precursor cells differentiated from the tonsil-derived mesenchymal stem cells exhibit higher expression of the neuron-specific marker Tuj1 as compared to precursor cells differentiated from mesenchymal stem cells derived from other tissues.

Specifically, the mesenchymal stem cells derived from other tissues include adipose-derived mesenchymal stem cells (AdMSC), bone marrow-derived mesenchymal stem cells (BM-MSC) and umbilical cord-derived or cord blood-derived mesenchymal stem cells (e.g., Wharton's jelly-derived mesenchymal stem cells (WJ-MSC)), although not being limited thereto. The precursor cells differentiated from the tonsil-derived mesenchymal stem cells exhibit higher expression of the neuron-specific marker Tuj1 by 10% or more, specifically 30% or more, as compared to the precursor cells differentiated from the mesenchymal stem cells derived from other tissues.

The differentiation method of the present disclosure further includes, before the step of inducing differentiation into motor neurons, a step of forming cell aggregates by culturing the tonsil-derived mesenchymal stem cells in a suspended state.

In the step of forming cell aggregates, a proliferation medium containing FBS, penicillin/streptomycin, β-mercaptoethanol and non-essential amino acids may be used. Specifically, the proliferation medium may contain 5-20% (w/v) FBS, 0.5-2% (w/v) penicillin/streptomycin, 0.05-0.2 mM β-mercaptoethanol and 0.5-2% (w/v) non-essential amino acids. Most specifically, it may contain 10% (w/v) FBS, 1% (w/v) penicillin/streptomycin, 0.1 mM β-mercaptoethanol and 1% (w/v) non-essential amino acids.

Among the ingredients contained in the proliferation medium, the non-essential amino acids are amino acids not synthesized metabolically in the body. Specifically, they may include one or more of glycine, L-alanine, L-aspartic acid, L-asparagine, L-glutamic acid, L-proline or L-serine, although not being limited thereto.

The proliferation medium used in the step described above may be one selected from DMEM (Dulbecco's modified Eagle's medium), RPMI1640 (Roswell Park Memorial Institute 1640), MEM (minimum essential medium) or Ham F10. Specifically, the medium may be DMEM (Dulbecco's modified Eagle's medium).

In the step described above, the cell aggregates may be formed by culturing 5x10⁶ to 7x10⁶ cells per 10 mL of a medium on a polyethyleneimine-coated culture dish in a suspended state for 1-7 days. The cell aggregates are formed to induce the differentiation into motor neuron more properly by enhancing interaction between the stem cells and forming a structure similar to that of an embryonic body.

The differentiation method of the present disclosure may further include, after the step of forming cell aggregates and before the step of inducing differentiation into motor neurons, a step of differentiating the formed cell aggregates into neural precursor cells by subculturing up to 1-3 passages.

In the present disclosure, subculturing refers to a method of culturing cells by replacing culture vessels or dividing a cell population in order to culture the stem cells for passages continuously in a health state. Each replacement of the culture vessel or division of the cell population is called one passage. In the present disclosure, the term passage may be used interchangeably with generation.

In another aspect, the present disclosure provides motor neurons prepared according to the method for differentiating into motor neurons described above.

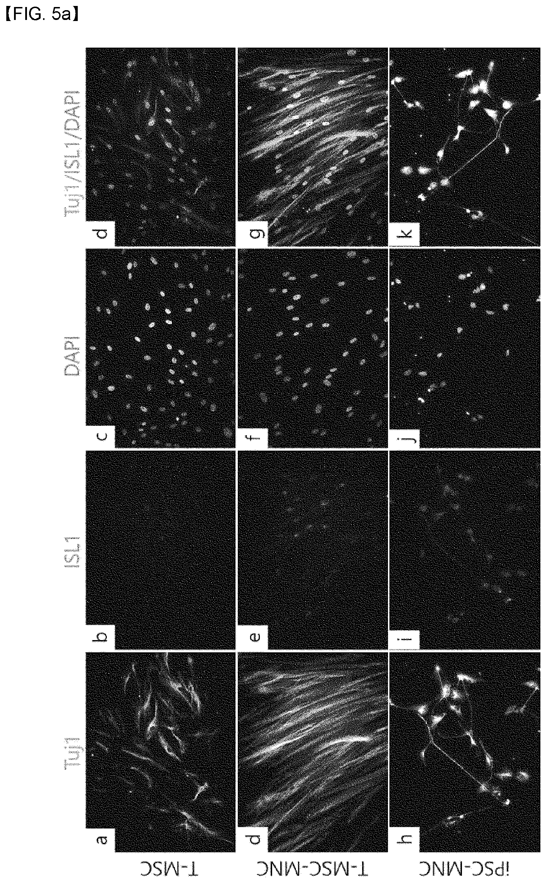

The motor neurons differentiated from the tonsil-derived mesenchymal stem cells of the present disclosure show difference in the intensity of expressed markers and the morphology of differentiated motor neurons from the motor neurons differentiated from other stem cells (FIGS. 5a-5c).

The motor neurons prepared according to the present disclosure exhibit increased expression of ISL1 (insulin gene enhancer protein), HB9 (homeobox protein) or ChAT (choline acetyltransferase).

ISL1 (insulin gene enhancer protein) is a factor involved in the generation and differentiation of motor neurons and is a representative marker of motor neurons.

HB9 (homeobox protein) is known to be expressed selectively in motor neurons of the central nervous system of vertebrates during development and have functions essential in establishing the post-mitotic identity of motoneurons. However, it is recently recognized as a marker essential for identifying differentiation and maturation from stem cells to motor neurons both in the central nervous system and the peripheral nervous system.

ChAT (choline acetyltransferase) is an enzyme which produces acetylcholine by attaching acetate ion bound to acetyl-CoA to choline. It is a representative marker of motor neurons.

Specifically, in an example of the present disclosure, the expression of ISL1, HB9 and ChAT in motor neurons prepared according to the present disclosure was analyzed by PCR, immunofluorescence assay and western blotting and it was confirmed that the motor neurons differentiated for 2 weeks or longer exhibit increased expression of ISL1, HB9 and ChAT, which are representative markers of motor neurons.

The motor neurons prepared according to the present disclosure exhibit increased secretion of acetylcholine. Additionally, the motor neurons are capable of forming a neuromuscular junction when co-cultured with skeletal muscle cells.

Acetylcholine is a neurotransmitter used at the neuromuscular junction, which is secreted from the synaptic vesicle at the axon terminal of the presynaptic neuron, passes through the synaptic cleft, and then transmits nerve signals by binding to the postsynaptic neuron. In an example of the present disclosure, it was confirmed that the motor neurons prepared according to the present disclosure exhibit increased secretion of acetylcholine. In addition, in an example of the present disclosure, it was confirmed that the motor neurons prepared according to the present disclosure exhibit expression of acetylcholine receptors when co-cultured with skeletal muscle cells.

The acetylcholine receptors are expressed to receive acetylcholine secreted from the motor neurons of the present disclosure. The motor neurons of the present disclosure can establish a normal nerve signal transmission system mediated by acetylcholine by forming a neuromuscular junction when co-cultured with skeletal muscle cells.

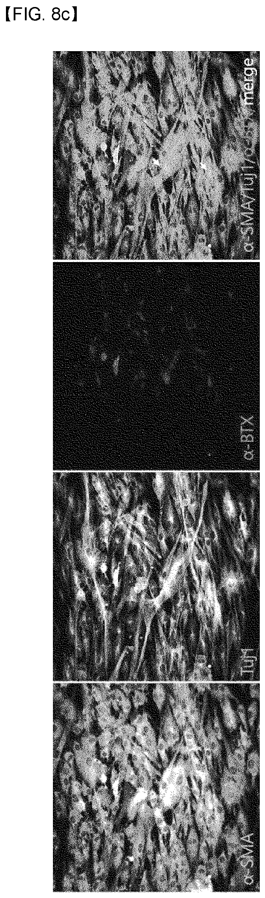

The neuromuscular junction is a chemical synapse formed between a motor neuron and a muscle fiber. It was confirmed that the motor neurons of the present disclosure differentiated from tonsil-derived mesenchymal stem cells form a neuromuscular junction as acetylcholine clusters are formed when they are co-cultured with skeletal muscle cells (SKMC) (see FIG. 8c). They are advantageous over the motor neurons differentiated from other mesenchymal stem cells in that motor neurons with more functionalities can be differentiated.

The motor neurons prepared according to the present disclosure can be subcultured up to 1-3 passages and can be used by thawing after freezing. Accordingly, the motor neurons of the present disclosure exhibit superior reproducibility even when subcultured and can be used as normal motor neurons even after storage for a long time.

In another aspect, the present disclosure provides a pharmaceutical composition for preventing or treating a neurological disorder, which contains the motor neurons according to the present disclosure as an active ingredient.

In another aspect, the present disclosure provides a cell therapy agent containing the motor neurons according to the present disclosure.

In another aspect, the present disclosure provides a pharmaceutical use of the composition described above for preventing or treating a neurological disorder.

In another aspect, the present disclosure provides a method for preventing or treating a neurological disorder, which includes a step of administering an effective amount of the motor neurons according to the present disclosure to a subject.

In the present disclosure, the prevention refers to any action of inhibiting a neurological disorder or delaying its progression by administering the composition of the present disclosure.

In the present disclosure, the treatment refers to any action of improving or favorably changing a neurological disorder by administering the composition of the present disclosure.

In the present disclosure, the subject refers to a mammal in need of the administration of the composition, and includes, specifically, human, companion animals such as dog, cat, etc., and livestock such as cow, pig, horse, sheep, etc.

In the present disclosure, the cell therapy agent refers to a medicine (regulated by the USFDA) used for the purpose of treatment, diagnosis or prevention with cells or tissues prepared by culturing and special manipulation after separation from a mammal, specifically a medicine used for the purpose of treatment, diagnosis or prevention by proliferating or screening autologous, allogeneic or heterologous living cells in vitro or otherwise changing the biological characteristics of the cells in order to restore the function of the cells or tissues.

The composition of the present disclosure may be used for preventing or treating a neurological disorder including damage to the central nervous system or the peripheral nervous system, degenerative brain disease, motor neuron disease, etc., specifically for preventing or treating motor neuron disease.

In the present disclosure, the motor neuron disease refers to a neurological disease and hereditary sensory neuropathy causing degenerative progress of motor neurons that control the action of voluntary muscles. Specifically, the motor neuron disease may be amyotrophic lateral sclerosis (ALS), myasthenia gravis (MG) Charcot-Marie-Tooth disease (CMT) or spinal muscular atrophy (SMA), although not being limited thereto.

In the present disclosure, the effective amount refers to the amount of an active ingredient or a pharmaceutical composition that elicits a biological or medicinal response sought in a tissue system, animal or human by a researcher, a veterinarian, a medicinal doctor or a clinician, and includes an amount that induces alleviation of the symptoms of the corresponding disease or disorder. It is obvious to those skilled in the art that the effective amount and number of administrations for the active ingredient of the present disclosure will change depending on the desired effect.

The composition may be administered by formulating into unit-dosage pharmaceutical preparations suitable for administration into the body according to a common method in the pharmaceutical field. The preparation may be administered in amounts effective for single or multiple administration. Formulations suitable for this purpose include parenteral preparations such as an injection, a spray, etc. In addition, the composition for treating motor neuron disease may contain a common pharmaceutically acceptable inert carrier. The active ingredient may be transplanted or administered according to a method commonly used in the art. Specifically, the active ingredient may be directly attached or transplanted to the disease site of a patient in need of treatment, although not being limited thereto. In addition, the administration may be carried out by non-surgical administration using a catheter or surgical administration such as injection, transplantation, etc. followed by incision of the disease site. An administration dosage of 1.0x10⁵ to 1.0x10⁸ cells/kg body weight, specifically 1.0x10⁶ to 1.0x10⁷ cells/kg body weight may be administered at once or multiple times. However, it should be understood that the actual administration dosage of the active ingredient is to be determined in consideration of various factors such as the disease to be treated, the severity of the disease, administration route, the body weight, age and sex of a patient, etc., and, therefore, the scope of the present disclosure is not limited by the administration dosage by any means.

### [Advantageous Effects]

A differentiation method of the present disclosure enables securing of a large quantity of motor neurons due to thigh differentiation potency into motor neurons. Since the cells which are differentiated according to the present disclosure are obtained using discarded tonsillar tissues, there exhibit high tissue compatibility and are highly applicable as a cell therapy agent.

### [Brief Description of Drawings]

FIG. 1 schematically shows induction of tonsil-derived mesenchymal stem cells (T-MSC, A) to neural precursor cells (NP, B) and differentiation into motor neurons (MN, C) as well as the ingredients of a differentiation medium and the morphology of the cells.
FIG. 2 shows that motor neurons differentiated by a method according to the present disclosure can proliferate normally when subcultured for 2 and 3 passages.
   MN2.5w: motor neurons differentiated for 2.5 weeks;
   p2: subculturing for 2 weeks; and
   p3: subculturing for 3 weeks (the same for FIG. 3).
FIG. 3 shows that motor neurons (MN) differentiated by a method according to the present disclosure can be used even after freezing and then thawing.
FIG. 4 shows a result of differentiating tonsil-derived mesenchymal stem cells (T-MSC) into motor neurons (MN) and confirming the increased expression of ISL1, HB9 and ChAT depending on differentiation period by real-time PCR.
   MN2w: motor neurons differentiated for 2 weeks;
   MN3w: motor neurons differentiated for 3 weeks; and
   MN4w: motor neurons differentiated for 4 weeks.
FIG. 5a shows a result of differentiating tonsil-derived mesenchymal stem cells into motor neurons for 2 weeks and confirming the increased expression of ISL1 in the cells by immunofluorescence staining.
FIG. 5b shows a result of differentiating tonsil-derived mesenchymal stem cells into motor neurons for 2 weeks and confirming the increased expression of HB9 in the cells by immunofluorescence staining.
FIG. 5c shows a result of differentiating tonsil-derived mesenchymal stem cells into motor neurons for 2 weeks and confirming the increased expression of ChAT in the cells by immunofluorescence staining.
FIGS. 6a-6d show a result of differentiating tonsil-derived mesenchymal stem cells into motor neurons for 4 weeks and investigating the change in the expression of ISL1 (FIG. 6b), HB9 (FIG. 6c) and ChAT (FIG. 6d) in the motor neurons depending on differentiation period by western blotting.
FIG. 7 shows a result of differentiating tonsil-derived mesenchymal stem cells into motor neurons and statistically comparing the concentration of acetylcholine in supernatants depending on differentiation period as percentage with respect to a differentiation medium.
   NPC: neural precursor cells;
   MNC2w: motor neurons differentiated for 2 weeks;
   MNC3w: motor neurons differentiated for 3 weeks; and
   MNC4w: motor neurons differentiated for 4 weeks.
FIG. 8a shows the optical microscopic images of tonsil-derived mesenchymal stem cells before and after differentiating into motor neurons and motor neurons being co-cultured with muscle cells.
FIG. 8b shows a result of co-culturing T-MSCs that have been differentiated for 2 weeks into motor neurons according to the present disclosure with human skeletal muscle cells and confirming that a neuromuscular junction can be formed by fluorescence immunostaining and α-BTX treatment (hSKMC: human skeletal muscle cells only; T-MSC-MNC: motor neurons derived from tonsil-derived stem cells only; hSKMC & T-MSC-MNC: motor neurons derived from tonsil-derived stem cells co-cultured with human skeletal muscle cells).
FIG. 8c shows a result of co-culturing T-MSCs differentiated into motor neurons with human skeletal muscle cells as in FIG. 8b and confirming the formation of a neuromuscular junction and the morphology of the two cells by staining with different proteins. α-SMA (α-smooth muscle actin, blue) shows the morphology of the muscle cells, Tuj1 (beta III tubulin, green) shows the morphology of the neurons, and α-BTX (bungarotoxin, red) shows acetylcholine receptor clusters at the neuromuscular junction. The three images are merged in the rightmost image.
FIG. 9 shows a result of differentiating tonsil-derived mesenchymal stem cells (T-MSC) into motor neurons (MNC) and confirming the increased expression of four neurotrophic factors in the cells by real-time PCR.
FIG. 10 shows a result of investigating the expression of vimentin in T-MSCs by immunofluorescence staining.
FIG. 11 shows a result of investigating the expression of Tuj1 in T-MSCs and neural precursor cells (NPCs) derived therefrom by immunofluorescence staining.

### [Best Mode]

Hereinafter, the present disclosure will be described in detail through examples. However, the following examples are for illustrative purposes only and the scope of the present disclosure is not limited by the examples.

### Example 1: Differentiation of tonsil-derived mesenchymal stem cells into motor neurons

### Example 1-1: Culturing of tonsil-derived mesenchymal stem cells

Tonsil-derived mesenchymal stem cells (TMSC) were obtained from the tonsillar tissues of patients who received tonsillectomy Department of Otorhinolaryngology-Head and Neck Surgery of Ewha Womans University Mokdong Hospital (tissues of young patients aged 4-20 years, approved by the Institutional Review Board: ECT 11-53-02). Stem cells were isolated and cultured in DMEM (Dulbecco's modified Eagle's medium, GIBCO) supplemented with 10% FBS (Hyclone), 1% penicillin/streptomycin (GIBCO), 0.1 mM β-mercaptoethanol (Sigma) and 1% non-essential amino acids (GIBCO).

### Example 1-2: Differentiation of tonsil-derived mesenchymal stem cells into motor neurons

The tonsil-derived mesenchymal stem cells were differentiated into motor neurons (MN) according to the following stages.

Spheres were formed as a first stage of inducing differentiation. The spheres were prepared by suspending 5,000,000-7,000,000 cells per 10 mL of the proliferation medium of Example 1 on a PEI-coated 100-mm Petri dish and inducing cell aggregation for 1-2 days. The formed spheres were replated onto a culture dish and differentiation into neural precursor cells (NPC) was induced by subculturing in a proliferation medium up to passage 1, 2 or 3.

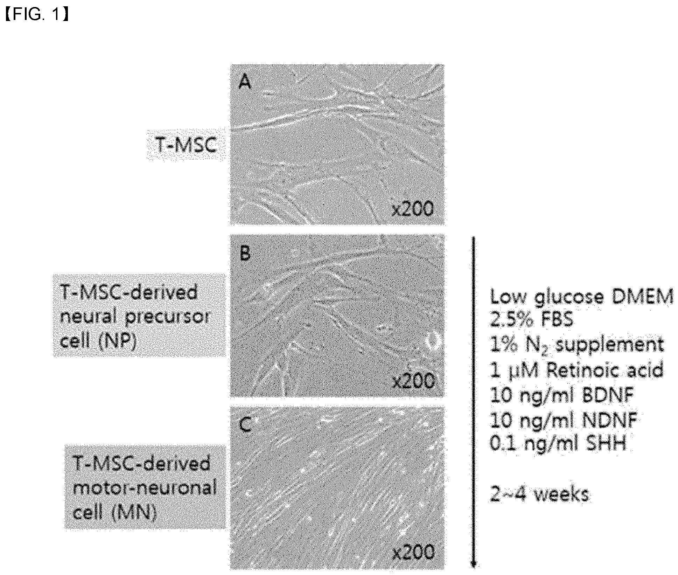

The differentiated neural precursor cells were cultured additionally in a differentiation medium [low-glucose DMEM, 2.5% FBS, 1% N₂ supplement, 1µM retinoic acid, 10 ng/mL brain-derived neurotrophic factor (BDNF), 10 ng/mL nerve growth factor (NGF), 0.1 ng/mL sonic hedgehog (SHH)] for 2-4 weeks. Through this, motor neurons were prepared (FIG. 1).

### Example 1-3: Subculturing of differentiated motor neurons

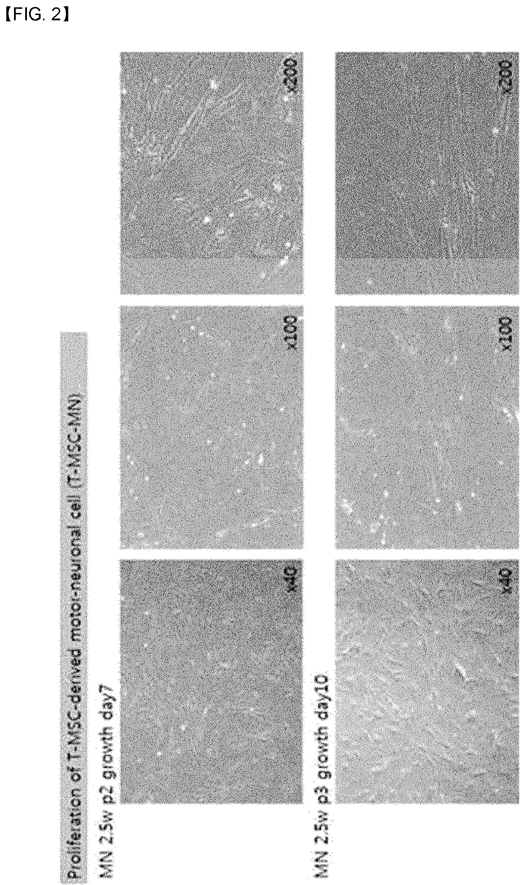

As a result of subculturing the differentiated motor neurons for 2.5 weeks, it was confirmed that the motor neurons subcultured for 2 and 3 passages have normal proliferation ability. Accordingly, it was confirmed that the motor neurons differentiated according to the present disclosure can proliferate normally even when subcultured (FIG. 2).

### Example 1-4: Use of differentiated motor neurons after freezing and thawing

Motor neurons differentiated for 2.5 weeks according to the method described above were frozen on day 10 after culturing and then cell morphology was observed after thawing on day 14. No change in morphology was observed even after the freezing and thawing.

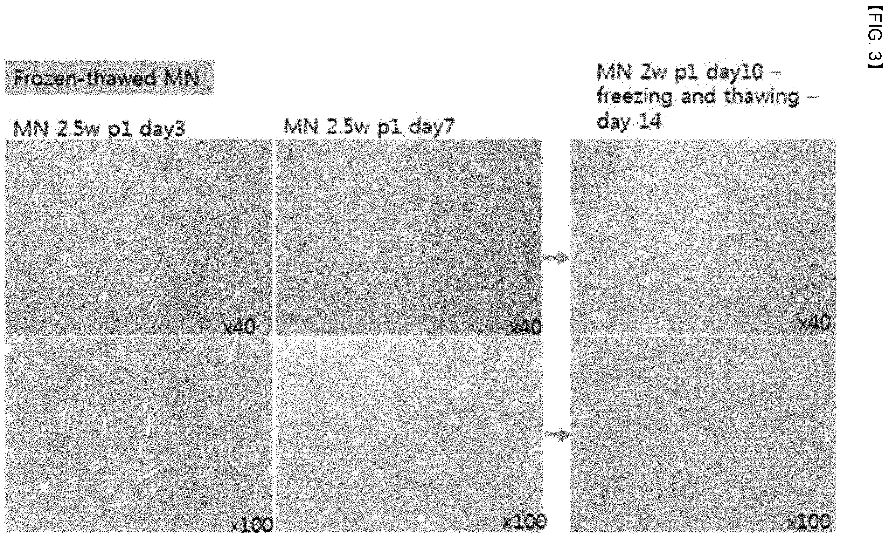

Accordingly, it was confirmed that the motor neurons differentiated according to the present disclosure can be used as normal motor neurons even after freezing and thawing (FIG. 3).

### Example 3: Investigation of differentiation potency from tonsil-derived mesenchymal stem cells into motor neurons by PCR

In order to investigate the differentiation potency from tonsil-derived mesenchymal stem cells into motor neurons, the expression level of ISL1 (insulin gene enhancer protein), HB9 and ChAT (choline acetyltransferase), which are representative markers of motor neurons, was analyzed by real-time PCR.

Total RNA was extracted using an RNeasy mini kit (Qiagen Inc.) according to the manufacturer's instructions. cDNA was synthesized using Superscript II (Invitrogen) and an oligo-d(T)20 primer by conducting reaction at 42 °C for 1 hour and at 72 °C for 15 minutes. For the cDNA, quantitative real-time PCR was performed using SYBR^{®} Premix Ex Taq^{™} kits (TaKaRa Bio Inc., Shiga, Japan) on an ABI 7500 fast real-time PCR system (Applied Biosystems/Thermo Fisher Scientific, Waltham, MA, USA). The relative expression level of the ISL1, HB9 and ChAT genes was calculated using the comparative Ct method (2^{-ΔΔCt}), and all measurements were carried out in triplicate.

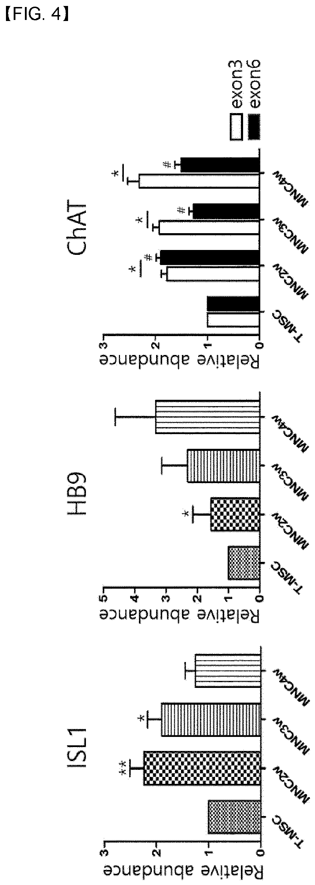

The result is shown in FIG. 4. As shown in FIG. 4, it was confirmed that, when tonsil-derived mesenchymal stem cells were differentiated into motor neurons, the expression of ISL1, HB9 and ChAT, which are markers of motor neurons, was increased from 2 weeks after the differentiation, which confirms the differentiation into motor neurons.

Specifically, ISL1 is a motor neuron-specific marker whose expression is increased during the early stage of differentiation into motor neurons. As shown in FIG. 4, the highest expression of ISL1 at 2 weeks after the differentiation means that the differentiation rate is the highest at 2 weeks after the differentiation, and the relatively decreased expression of ISL1 at 3 weeks as compared to 2 weeks after the differentiation means that differentiation into motor neurons has proceeded already. Statistically significant increased expression of ISL1 as compared to undifferentiated T-MSCs cells was observed at 2 weeks and 3 weeks. HB9 is also a motor neuron-specific marker whose expression is increased during the early stage of differentiation into motor neurons. Although the expression of HB9 was increased gradually with differentiation period, statistically significant expression as compared to undifferentiated T-MSCs cells was observed only at 2 weeks.

ChAT is a motor neuron marker whose expression is increased as differentiation proceeds, whereas the expression of ISL1 is increased during the early stage of differentiation, and is called an acetylcholinergic neuron marker. There exist two isoforms of ChAT: common type ChAT (cChAT) present in both the central nervous system and the peripheral nervous system; and peripheral type ChAT (pChAT) preferentially expressed in the peripheral nervous system. As shown in FIG. 4, the expression of exon 3 of ChAT was significantly expressed between 2 weeks and 4 weeks after the differentiation, showing the characteristics of both the central nervous system and the peripheral nervous system. The expression of exon 6 was increased at 2 weeks after the differentiation and then was decreased relatively from 3 weeks. This is due to skipping from exon 6 to exon 9 during post-transcriptional modification, suggesting that the characteristics of the peripheral nervous system become dominant as the differentiation proceeds. The above results for ChAT suggest that the differentiation into motor neurons begins at 2 weeks after the differentiation and proceeds until 3 weeks and 4 weeks.

Through these experimental results, it was confirmed that the cells differentiated from tonsil-derived mesenchymal stem cells exhibit the characteristics of motor neurons. Accordingly, it was confirmed that the differentiation medium of the present disclosure exhibits superior differentiation potency into motor neurons.

### Example 4: Investigation of differentiation potency from tonsil-derived mesenchymal stem cells into motor neurons by immunofluorescence assay

The differentiation potency into motor neurons was investigated by immunofluorescence staining. After differentiating tonsil-derived mesenchymal stem cells for 2 weeks, motor neurons were cultured on a cover slip. After the differentiation was finished, the cells were fixed in a 4% paraformaldehyde solution for 15 minutes at room temperature and then washed with PBS. The washed cells were treated in a PBS solution with 0.1% Tween-20 and 2% bovine serum albumin added for 1 hour and diluted with antibodies for detection of differentiation at a ratio designated by the producer. After addition to PBS, incubation was conducted at room temperature for 1 hour or overnight at low temperature. Subsequently, after washing again with PBS, the cells were treated with TRITC (tetrarhodamine isothiocyanate)- or FITC (fluorescein isothiocyanate)-conjugated secondary antibodies at room temperature or low temperature in the same manner as the primary antibodies. A mounting solution (Vectashield) with DAPI added was used for contrast staining of cell nuclei. After mounting, the cells were observed using a fluorescence microscope.

As can be seen from FIG. 5a, whereas T-MSCs showed no red fluorescence signal of ISL1 at all, the differentiated motor neurons (T-MSC-MNC) showed strong red fluorescence signal of ISL1 (b and e of FIG. 5a). In addition, it was confirmed that the expression of class III beta-tubulin (Tuj1), which is a neuron-specific protein, is increased as the differentiation proceeds (a and d of FIG. 5a). In addition, in order to verify the adequacy of the method for differentiating into motor neurons by immunofluorescence assay, induced pluripotent stem cell-derived motor neurons (iXCell^{™} human iPSC-derived motor neurons, iPSC-MNC) were purchased and the expression of ISL1 and Tuj1 was observed. As a result, the expression pattern of the two markers (ISL1 and Tuj1) was identical although T-MSC-MNCs and iPSC-MNCs showed slightly different cell morphologies (h, i, j and k of FIG. 5a).

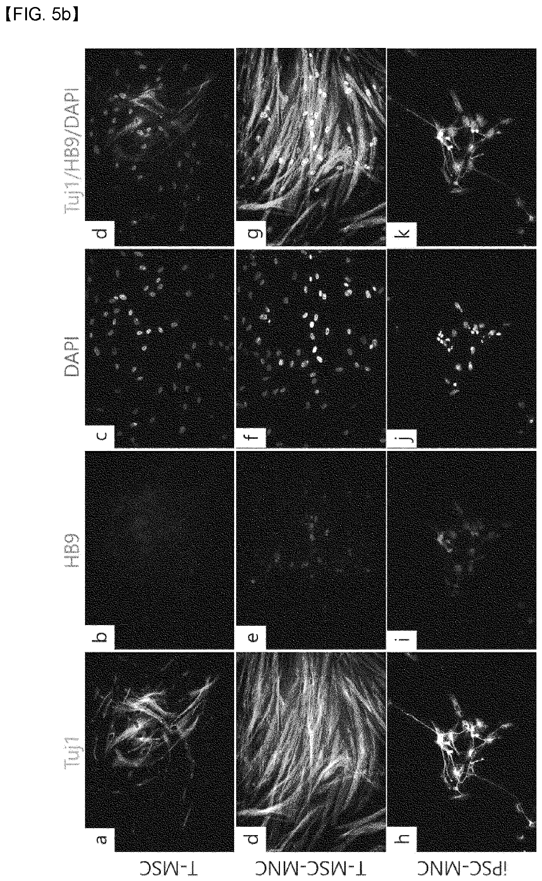

As seen from FIG. 5b, whereas T-MSCs showed no red fluorescence signal of HB9 at all, the differentiated motor neurons (T-MSC-MNC) showed strong red fluorescence signal of HB9 (b and e of FIG. 5b). In addition, it was confirmed that the expression of Tuj1 is increased as the differentiation proceeds (a and d of FIG. 5b). In addition, the expression of HB9 and Tuj1 in iPSC-MNCs was observed. As a result, the expression pattern of the two markers (HB9 and Tuj1) was identical although T-MSC-MNCs and iPSC-MNCs showed slightly different cell morphologies (h, i, j and k of FIG. 5b).

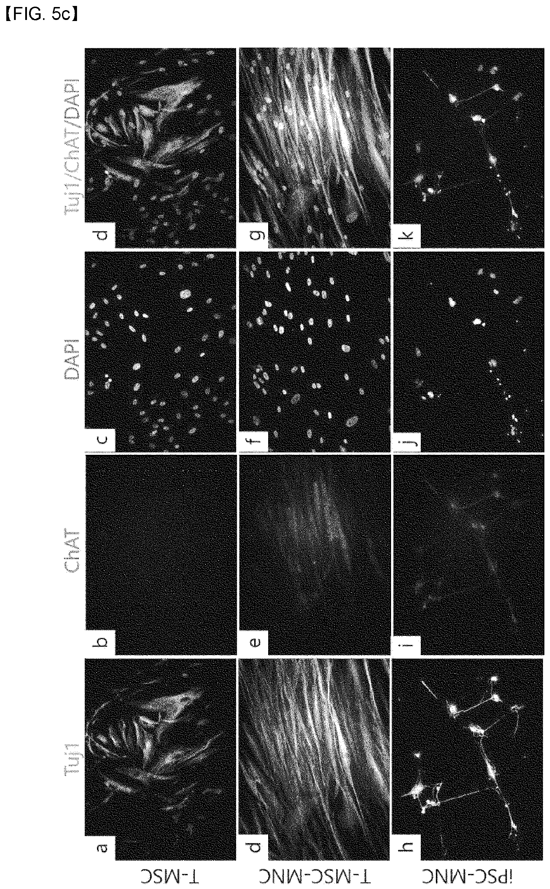

As seen from FIG. 5c, whereas T-MSCs showed no red fluorescence signal of ChAT at all, the differentiated motor neurons (T-MSC-MNC) showed strong red fluorescence signal of ChAT (b and e of FIG. 5c). In addition, it was confirmed that the expression of Tuj1 is increased as the differentiation proceeds (a and d of FIG. 5c). In addition, the expression of ChAT and Tuj1 in iPSC-MNCs was observed. As a result, the expression pattern of the two markers (ChAT and Tuj1) was identical although T-MSC-MNCs and iPSC-MNCs showed slightly different cell morphologies (h, i, j and k of FIG. 5c).

Through these experimental results, it was confirmed that the cells differentiated from tonsil-derived mesenchymal stem cells have the characteristics of motor neurons. Accordingly, it was confirmed that the differentiation medium of the present disclosure exhibits superior differentiation potency into motor neurons.

### Example 5: Investigation of differentiation potency from tonsil-derived mesenchymal stem cells into motor neurons by western blotting

The differentiation from tonsil-derived mesenchymal stem cells into motor neurons was investigated by western blotting.

Tonsil-derived mesenchymal stem cells and cells in different stages of differentiation (undifferentiated tonsil-derived mesenchymal stem cells, neural precursor cells, and motor neurons differentiated for 2 to 4 weeks) were lysed by adding to a lysis buffer containing a protease inhibitor (Roche). Total proteins (10-30 µg) were immunoblotted with primary antibodies (ISL1, HB9, ChAT), and GAPDH (Abcam) was used as an internal control. Band intensity was quantified using LAS-3000 (Fuji Film) and normalized to the intensity of GAPDH.

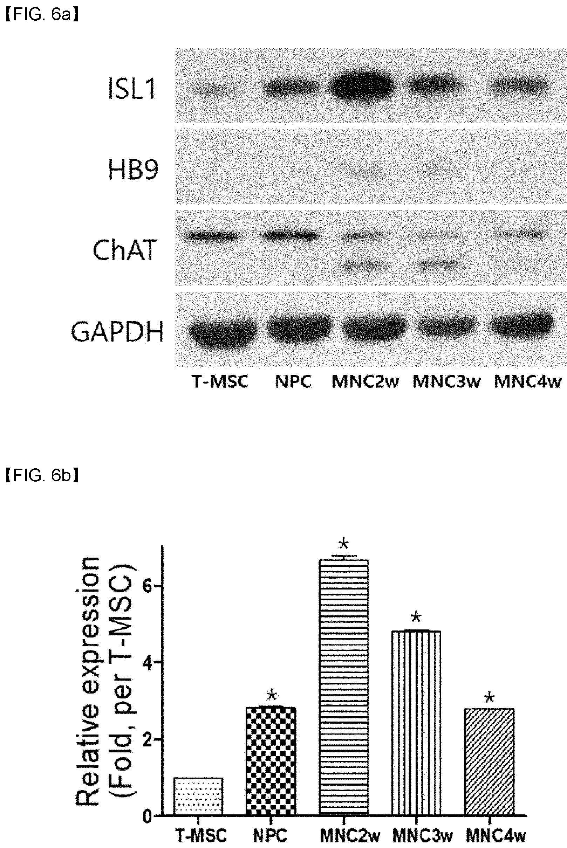

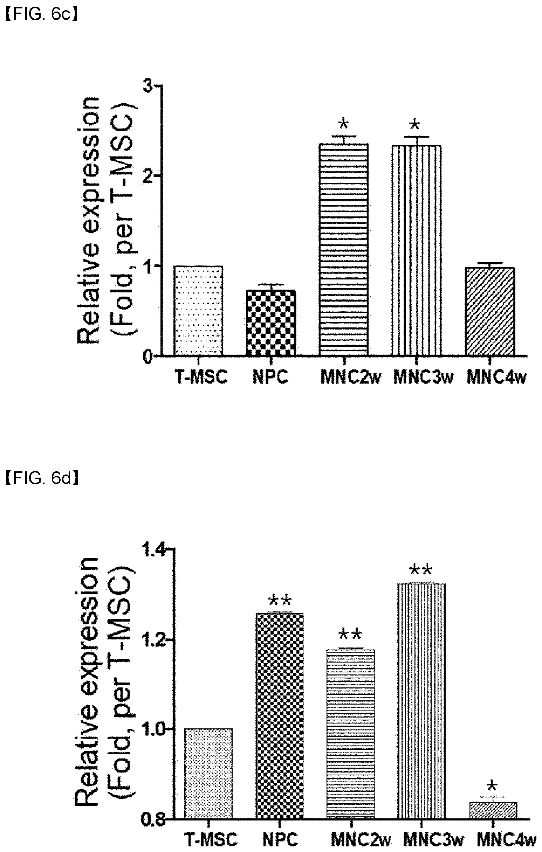

The result is shown in FIGS. 6a-6d. The band intensities of FIG. 6a are plotted in FIG. 6b (ISL1), FIG. 6c (HB9) and FIG. 6d (ChAT). As can be seen from FIGS. 6a-6d, although the ISL1 protein was expressed slightly in T-MSCs, the expression was increased as the cells were differentiated into neural precursor cells (NPC) and reached maximum at 2 weeks after the differentiation (FIG. 6b). The HB9 protein was hardly expressed in T-MSCs and NPCs, and the expression was increased at 2 weeks and 3 weeks after the differentiation (FIG. 6c). The isotype 2 protein of ChAT showed two bands at 2 weeks and 3 weeks after the differentiation, confirming differentiation into motor neurons (FIG. 6d).

Similarly to Example 3, the increased expression of isotype 2 in motor neurons 2 weeks after the differentiation means that the differentiated motor neurons exhibit the characteristics of peripheral nerves.

Through these experimental results, it was confirmed that the cells differentiated from tonsil-derived mesenchymal stem cells have the characteristics of motor neurons. Accordingly, it was confirmed that the differentiation medium of the present disclosure exhibits superior differentiation potency into motor neurons.

### Example 6: Confirmation of differentiation potency into motor neurons from increase in acetylcholine

For a supernatant (or conditioned medium) taken from a culture dish in which tonsil-derived mesenchymal stem cells were being differentiated into motor neurons for 4 weeks and a differentiation medium, the increase in acetylcholine with respect to the differentiation medium was calculated as percentage using an acetylcholine assay kit (Fluorometric; Cell Biolabs, INC. CA, USA).

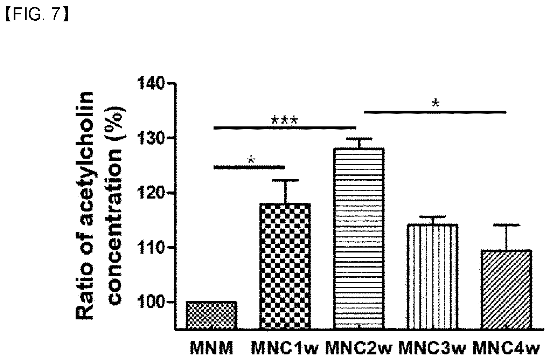

The result is shown in FIG. 7. As can be seen from FIG. 7, when T-MSCs were differentiated into T-MSC-MNCs, the secretion of acetylcholine began to increase from 1 week after the differentiation and reached maximum at 2 weeks. This result was statistically significant when repeated three times. This means that, when tonsil-derived mesenchymal stem cells are differentiated into motor neurons, the highest differentiation rate is achieved at 2 weeks after the differentiation.

Acetylcholine is a neurotransmitter of the neuromuscular junction secreted at the axon terminal. The increased secretion of acetylcholine in the motor neurons prepared according to the present disclosure means that they can function as normal motor neurons.

Through this, it was confirmed that tonsil-derived mesenchymal stem cells are differentiated into motor neurons when cultured using the differentiation medium of the present disclosure.

### Example 7: Neuromuscular junction forming-ability of differentiated motor neurons

It was investigated whether a neuromuscular junction is formed in order to investigate whether the motor neurons differentiated according to the present disclosure actually exhibit the characteristics of motor neurons.

Specifically, motor neurons differentiated from tonsil-derived mesenchymal stem cells for 2 weeks were co-cultured with human skeletal muscle cells (hSKMC) and fixed 4-5 days later. Then, it was investigated whether the cells are neurons by staining with Tui1 (green) by fluorescence immunostaining, and the presence of acetylcholine receptors was investigated by treating with Alexa 555-conjugated α-BTX to confirm the formation of the neuromuscular junction.

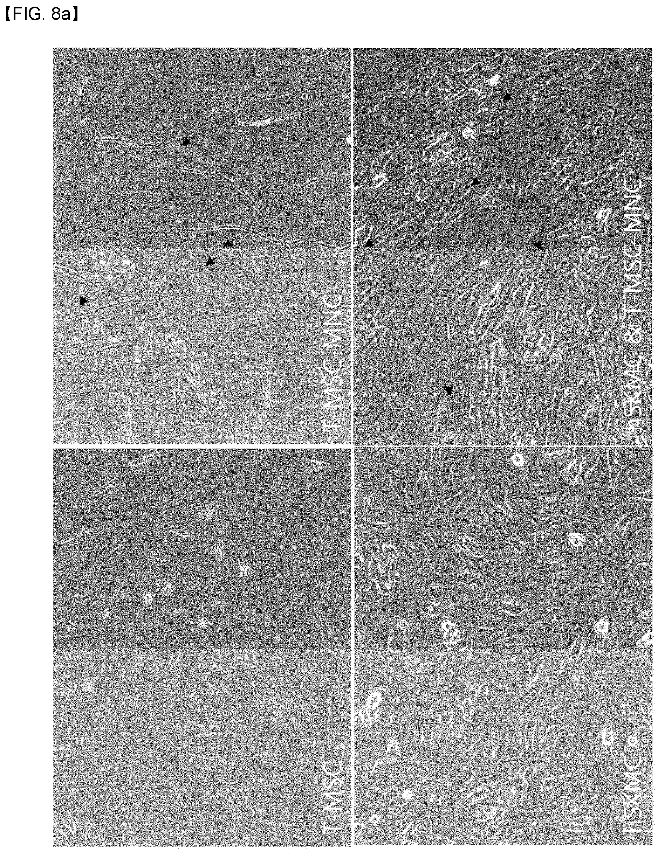

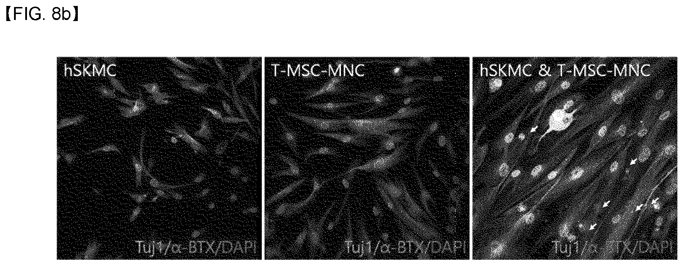

The result is shown in FIG. 8. First, the morphological change of T-MSC-MNCs was observed before investigating the formation of the neuromuscular junction (FIG. 8a). Compared with T-MSCs, T-MSC-MNCs became multipolar and the length of the cell body was increased like typical motor neurons (arrows in FIG. 8a). In addition, the change in the cell morphology of hSKMCs being co-cultured could be observed as well as the cellular characteristics of hSKMCs and T-MSC-MNCs being co-cultured.

As seen from FIG. 8b, when T-MSCs or hSKMCs were cultured alone, no red fluorescence was observed at all and the expression of Tuj1 was low. In contrast, when the motor neurons differentiated according to the present disclosure were co-cultured with skeletal muscle cells, red fluorescence was observed and the expression of Tuj1 was increased. In order to investigate the formation of the neuromuscular junction in more detail, triple staining was performed with the muscle-specific marker α-smooth muscle actin (α-SMA) and the neuron-specific markers Tuj1 and α-BTX (FIG. 8c). As a result, the presence of red acetylcholine receptors (arrows) was clearly observed when the two cells were co-cultured.

This result suggests that the cells differentiated from tonsil-derived mesenchymal stem cells for 2 weeks have the possibility of signaling through the junction with skeletal muscle cells, which is the most important function of motor neurons.

The red fluorescence indicates the presence of acetylcholine receptors in the motor neurons co-cultured with the skeletal muscle cells. A normal nerve signal transmission system mediated by acetylcholine can be established based on this experimental result because the motor neurons differentiated according to the present disclosure are capable of forming the neuromuscular junction.

### Example 8: Investigation of increase of neurotrophic factors in motor neurons differentiated from tonsil-derived mesenchymal stem cells by PCR

In order to investigate characterization of the motor neurons differentiated from tonsil-derived mesenchymal stem cells, the change in the expression of neurotrophic factors such as brain derived neurotrophic factor (BDNF), glial cell-derived neurotrophic factor (GDNF), nerve growth factor (NGF) and heregulin (HRG), which promote initial growth and development of neurons in the central nervous system and the peripheral nervous system, was analyzed by real-time PCR. Total RNA was extracted using an RNeasy mini kit (Qiagen Inc.) according to the manufacturer's instructions. cDNA was synthesized using Superscript II (Invitrogen) and an oligo-d(T)20 primer by conducting reaction at 42 °C for 1 hour and at 72 °C for 15 minutes. For the cDNA, quantitative real-time PCR was performed using SYBR^{®} Premix Ex Taq^{™} kits (TaKaRa Bio Inc., Shiga, Japan) on an ABI 7500 fast real-time PCR system (Applied Biosystems/Thermo Fisher Scientific, Waltham, MA, USA). The relative expression level of the BDNF, GDNF, NGF and HRG genes was calculated using the comparative Ct method (2^{-ΔΔCt}), and all measurements were carried out in triplicate.

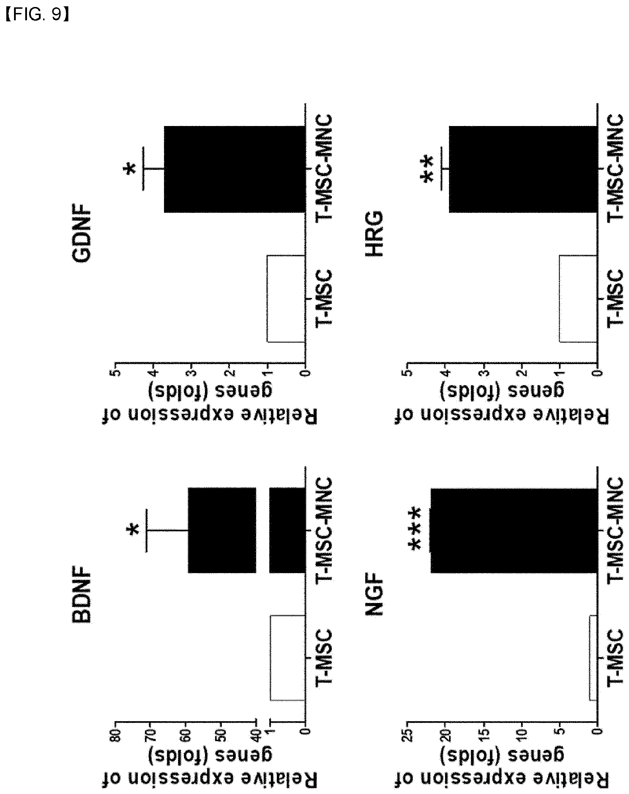

As shown in FIG. 9, the expression of the four neurotrophic factors was increased statically significantly after differentiation into T-MSC-MNCs. In particular, it is to be noted that the expression of BDNF, GDNF and HRG, which are nerve growth factors not added to the differentiation medium, was increased significantly.

### Example 10: Comparison with AdMSCs, BMMSCs and WJ-MSCs

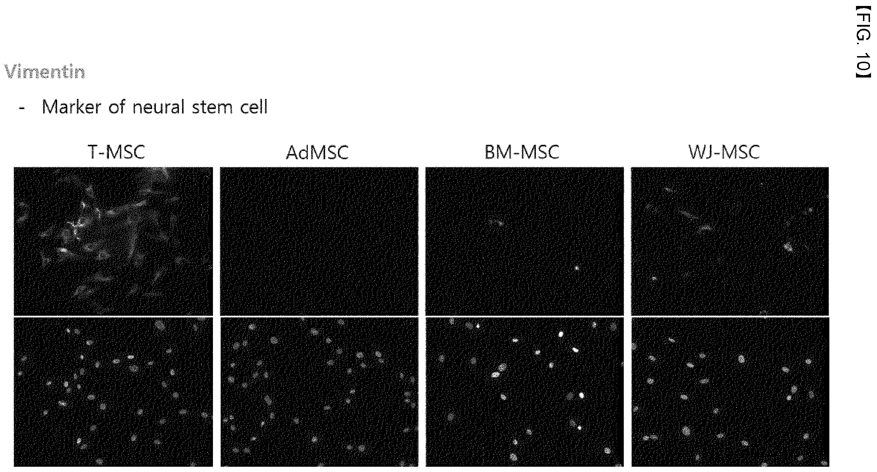

FIG. 10 shows a result of investigating the expression of vimentin in T-MSCs by immunofluorescence staining. Vimentin is a protein often used as a neural precursor cell marker. From FIG. 10, it can be seen that the T-MSCs have remarkably higher differentiation potency into motor neurons as compared to other MSCs (AdMACs, BM-MSCs and WJ-MSCs).

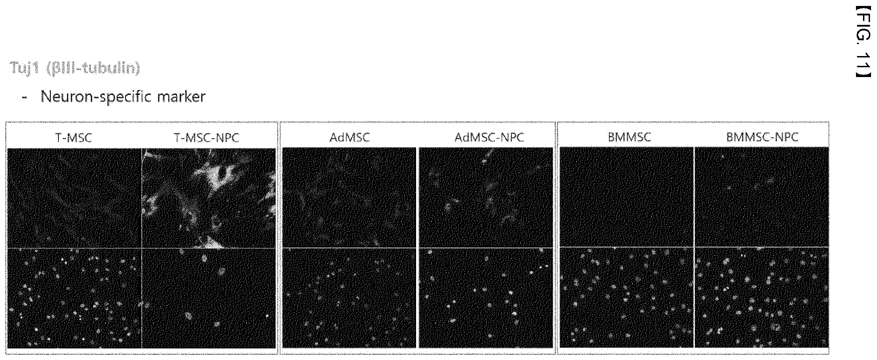

FIG. 11 shows a result of investigating the expression of Tuj1 in T-MSCs and neural precursor cells (NPCs) derived therefrom by immunofluorescence staining. From FIG. 11, it can be estimated that the neural precursor cells differentiated from the T-MSCs have remarkably higher differentiation potency into motor neurons as compared to the NPCs derived from other MSCs (AdMSCs and BM-MSCs) because the expression level of the neuron-specific marker Tuj1 is very high.

In the present specification, detailed description of the contents that can be fully recognized and inferred by those of ordinary skill in the art to which the present disclosure belongs was omitted.

## Claims

1. A differentiation medium composition suitable for differentiating tonsil-derived mesenchymal stem cells or neural precursor cells differentiated therefrom into motor neurons, comprising low-glucose DMEM (Dulbecco's modified Eagle's medium), FBS, N₂ supplement, retinoic acid, brain-derived neurotrophic factor (BDNF), nerve growth factor (NGF) and sonic hedgehog (SHH).

2. The differentiation medium composition according to claim 1, wherein the differentiation medium comprises low-glucose DMEM, 0.25-25% (w/v) FBS, 0.1-10% (w/v) N₂ supplement, 0.1-10 µM retinoic acid, 1-100 ng/mL brain-derived neurotrophic factor (BDNF), 1-100 ng/mL nerve growth factor (NGF) and 0.01-1 ng/mL sonic hedgehog (SHH).

3. A method for differentiating tonsil-derived mesenchymal stem cells or neural precursor cells into motor neurons, comprising a step of inducing differentiation into motor neurons by culturing tonsil-derived mesenchymal stem cells or neural precursor cells differentiated therefrom in the differentiation medium composition according to claim 1, wherein, in case of differentiating tonsil-derived mesenchymal stem cells into motor neurons, the method further comprises culturing the tonsil-derived mesenchymal stem cells in a suspended state to form cell aggregates and a step of differentiating the formed cell aggregates into neural precursor cells by subculturing up to 1 -3 passages.

4. The differentiation method according to claim 3, wherein the culturing is performed for 2-4 weeks.

5. The differentiation method according to claim 3, wherein, in the step of forming cell aggregates, a proliferation medium comprising FBS, penicillin/streptomycin, β-mercaptoethanol and non-essential amino acids is used.

6. The differentiation method according to claim 5, wherein the proliferation medium of the step of forming cell aggregates is DMEM (Dulbecco's modified Eagle's medium) comprising 5-20% (w/v) FBS, 0.5-2% (w/v) penicillin/streptomycin, 0.05-0.2 mM β-mercaptoethanol and 0.5-2% (w/v) non-essential amino acids.

7. The differentiation method according to claim 3, wherein the cell aggregates are formed by culturing 5x10⁶ to 7x10⁶ cells per 10 mL of a medium on a polyethyleneimine-coated culture dish in a suspended state for 1-7 days.

8. The differentiation method according to claim 3, wherein, the differentiation method further comprises a step of differentiating the cell aggregates into neural precursor cells by subculturing up to 1-3 passages.

9. The differentiation method according to claim 3, wherein the precursor cells are neural precursor cells.

10. The differentiation method according to claim 3, wherein the tonsil-derived mesenchymal stem cells exhibit higher expression of the neural precursor cell marker vimentin as compared to mesenchymal stem cells derived from other tissues.

11. The differentiation method according to claim 3, wherein the precursor cells differentiated from the tonsil-derived mesenchymal stem cells exhibit higher expression of the neuron-specific marker Tuj1 as compared to precursor cells differentiated from mesenchymal stem cells derived from other tissues.

## Patentansprüche

1. Differenzierungsmediumzusammensetzung, geeignet zum Differenzieren mesenchymaler Stammzellen der Tonsillen oder daraus differenzierter neuronaler Vorläuferzellen in Motoneuronen, umfassend DMEM (Dulbecco's Modified Eagle's Medium) mit geringem Glucosegehalt, FBS, N₂-Supplement, Retinsäure, zerebralen neurotrophen Faktor (BDNF), Nervenwachstumsfaktor (NGF) und Sonic Hedgehog (SHH).

2. Differenzierungsmediumzusammensetzung nach Anspruch 1, wobei das Differenzierungsmedium DMEM mit geringem Glucosegehalt, 0,25-25 % (Gew./Vol.) FBS, 0,1-10 % (Gew./Vol.) N₂-Supplement, 0,1-10 µM Retinsäure, 1-100 ng/ml zerebralen neurotrophen Faktor (BDNF), 1-100 ng/ml Nervenwachstumsfaktor (NGF) und 0,01-1 ng/ml Sonic Hedgehog (SHH) umfasst.

3. Verfahren zur Differenzierung von mesenchymalen Stammzellen der Tonsillen oder neuralen Vorläuferzellen in Motoneuronen, umfassend einen Schritt des Induzierens der Differenzierung in Motoneuronen durch Kultivierung von mesenchymalen Stammzellen der Tonsillen oder daraus differenzierten neuronalen Vorläuferzellen in der Differenzierungsmediumzusammensetzung nach Anspruch 1, wobei das Verfahren im Fall der Differenzierung von mesenchymalen Stammzellen der Tonsillen in Motoneuronen ferner die Kultivierung der mesenchymalen Stammzellen der Tonsillen in gelöstem Zustand zur Bildung von Zellaggregaten und einen Schritt der Differenzierung der gebildeten Zellaggregate in neuronale Vorläuferzellen durch Subkultivierung in bis zu 1-3 Passagen umfasst.

4. Differenzierungsverfahren nach Anspruch 3, wobei die Kultivierung für 2-4 Wochen durchgeführt wird.

5. Differenzierungsverfahren nach Anspruch 3, wobei im Schritt der Bildung von Zellaggregaten ein Proliferationsmedium verwendet wird, umfassend FBS, Penicillin/Streptomycin, β-Mercaptoethanol und nicht-essenzielle Aminosäuren.

6. Differenzierungsverfahren nach Anspruch 5, wobei das Proliferationsmedium des Schritts der Bildung von Zellaggregaten DMEM (Dulbecco's Modified Eagle's Medium) ist, umfassend 5-20 %(Gew./Vol.) FBS, 0,5-2 % (Gew./Vol.) Penicillin/Streptomycin, 0,05-0,2 mM β-Mercaptoethanol und 0,5-2 % (Gew./Vol.) nicht-essenzielle Aminosäuren.

7. Differenzierungsverfahren nach Anspruch 3, wobei die Zellaggregate durch Kultivierung von 5x10⁶ bis 7x10⁶ Zellen pro 10 ml eines Mediums auf einer mit Polyethylenimin beschichteten Kulturschale in gelöstem Zustand für 1-7 Tage gebildet werden.

8. Differenzierungsverfahren nach Anspruch 3, wobei das Differenzierungsverfahren ferner einen Schritt des Differenzierens der Zellaggregate in neuronale Vorläuferzellen durch Subkultivierung in bis zu 1-3 Passagen umfasst.

9. Differenzierungsverfahren nach Anspruch 3, wobei die Vorläuferzellen neuronale Vorläuferzellen sind.

10. Differenzierungsverfahren nach Anspruch 3, wobei die mesenchymalen Stammzellen der Tonsillen im Vergleich zu mesenchymalen Stammzellen aus anderen Geweben eine höhere Expression des neuronalen Vorläuferzellenmarkers Vimentin aufweisen.

11. Differenzierungsverfahren nach Anspruch 3, wobei die aus den mesenchymalen Stammzellen der Tonsillen differenzierten Vorläuferzellen im Vergleich zu aus mesenchymalen Stammzellen aus anderen Geweben differenzierten Vorläuferzellen eine höhere Expression des neuronenspezifischen Markers Tuj1 aufweisen.

## Revendications

1. Composition de milieu de différenciation convenant pour différencier des cellules souches mésenchymateuses dérivées de tonsille ou des cellules précurseurs neurales différenciées à partir de celles-ci en motoneurones, comprenant du DMEM (milieu d'Eagle modifié par Dulbecco) à faible teneur en glucose, du SVF, un supplément de N₂, de l'acide rétinoïque, du facteur neurotrophique dérivé du cerveau (BDNF), du facteur de croissance des nerfs (NGF) et de la protéine Sonic Hedgehog (SHH).

2. Composition de milieu de différenciation selon la revendication 1, dans laquelle le milieu de différenciation comprend du DMEM à faible teneur en glucose, 0,25 à 25 % (p/v) de SVF, 0,1 à 10 % (p/v) de supplément de N₂, de l'acide rétinoïque 0,1 à 10 µM, 1 à 100 ng/ml de facteur neurotrophique dérivé du cerveau (BDNF), 1 à 100 ng/ml de facteur de croissance des nerfs (NGF) et 0,01 à 1 ng/ml de protéine Sonic Hedgehog (SHH).

3. Procédé pour différencier des cellules souches mésenchymateuses dérivées de tonsille ou des cellules précurseurs neurales en motoneurones, comprenant une étape d'induction d'une différenciation en motoneurones par culture de cellules souches mésenchymateuses dérivées de tonsille ou de cellules précurseurs neurales différenciées à partir de celles-ci dans la composition de milieu de différenciation selon la revendication 1, dans lequel, dans le cas d'une différenciation de cellules souches mésenchymateuses dérivées de tonsille en motoneurones, le procédé comprend en outre la culture des cellules souches mésenchymateuses dérivées de tonsille dans un état en suspension pour former des agrégats cellulaires et une étape de différenciation des agrégats cellulaires formés en cellules précurseurs neurales par sous-culture sur jusqu'à 1 à 3 passages.

4. Procédé de différenciation selon la revendication 3, dans lequel la culture est effectuée pendant 2 à 4 semaines.

5. Procédé de différenciation selon la revendication 3, dans lequel, dans l'étape de formation d'agrégats cellulaires, un milieu de prolifération comprenant du SVF, de la pénicilline/streptomycine, du β-mercaptoéthanol et des acides aminés non essentiels est utilisé.

6. Procédé de différenciation selon la revendication 5, dans lequel le milieu de prolifération de l'étape de formation d'agrégats cellulaires est du DMEM (milieu d'Eagle modifié par Dulbecco) comprenant 5 à 20 % (p/v) de SVF, 0,5 à 2 % (p/v) de pénicilline/streptomycine, du β-mercaptoéthanol 0,05 à 0,2 mM, et 0,5 à 2 % (p/v) d'acides aminés non essentiels.

7. Procédé de différenciation selon la revendication 3, dans lequel les agrégats cellulaires sont formés par culture de 5 x 10⁶ à 7 x 10⁶ cellules par 10 ml d'un milieu sur une boîte de culture revêtue de polyéthylène-imine dans un état en suspension pendant 1 à 7 jours.

8. Procédé de différenciation selon la revendication 3, lequel procédé de différenciation comprend en outre une étape de différenciation des agrégats cellulaires en cellules précurseurs neurales par sous-culture sur jusqu'à 1 à 3 passages.

9. Procédé de différenciation selon la revendication 3, dans lequel les cellules précurseurs sont des cellules précurseurs neurales.

10. Procédé de différenciation selon la revendication 3, dans lequel les cellules souches mésenchymateuses dérivées de tonsille présentent une expression du marqueur de cellules précurseurs neurales vimentine supérieure à celle de cellules souches mésenchymateuses dérivées d'autres tissus.

11. Procédé de différenciation selon la revendication 3, dans lequel les cellules précurseurs différenciées à partir des cellules souches mésenchymateuses dérivées de tonsille présentent une expression du marqueur spécifique des neurones Tuj1 supérieur à celle de cellules précurseurs différenciées à partir de cellules souches mésenchymateuses dérivées d'autres tissus.
